# EUROPÄISCHE PATENTANMELDUNG

(11) **EP 1 743 608 A2**
(43) Veröffentlichungstag der Anmeldung: **17.01.2007**
(21) Anmeldenummer: 06014022.5
(22) Anmeldetag: 06.07.2006
(51) Int. Cl.: A61F 5/02

(54) **Rückenorthese**

(30) Priorität: 15.07.2005 DE 202005011650 U
(71) Anmelder: FERD. HAUBER GmbH & CO. KG, 72622 Nürtingen (DE)
(72) Erfinder: Sevim, Kaja, Dr. med., 31542 Bad Nenndorf (DE); Fahsing, Michael, 31655 Stadthagen (DE)
(74) Vertreter: Fuhlendorf, Jörn

(57) **Zusammenfassung**

Eine Rückenorthese (10) ist mit einem sich über einen wesentlichen Teil der Wirbelsäule, erstreckenden Stützteil (11) sowie mit einem Abdominalteil (13) und mit einer Gurtanordnung (12), deren obere Gurtabschnitte mit einem oberen und einem mittleren Bereich des Stützteils (11) verbunden und rucksackträgerartig geführt sind, versehen. Um zu erreichen, dass die Rückenorthese (10) einen gewissen Bewegungsumfang in Beugerichtung des Rumpfes zulässt sowie eine angemessene Abdominalstützung bewirkt und dennoch über eine längere Tragedauer von mehreren Stunden nicht wesentlich am Körper aus ihrer ursprünglichen Position verrutscht, ist vorgesehen, dass der Abdominalteil zu einer den gesamten unteren Körperteilumfang umschließenden Bandage (13) verlängert ist und dass das wirbelsäulenseitige Stützteil (11) an seinem unteren Ende entweder mit der Bandage (13) in Richtung der Wirbelsäule vertikal beweglich verbunden oder mit der Bandage (13) fest verbunden und in sich teleskopisch vertikal beweglich ist.

## Beschreibung

Die vorliegende Erfindung bezieht sich auf eine Rückenorthese nach dem Oberbegriff des Anspruchs 1.

Bei einer derartigen aus DE 297 20 475 U1 bekannten Rückenorthese ist der Stützteil nach Art einer relativ breiten Schiene ausgebildet, die über eine Gurtanordnung über ihre gesamte Länge hinweg längs der Wirbelsäule am Oberkörper hält. Hierzu besitzt die Gurtanordnung obere Gurtabschnitte, die in der Art von Rucksackträgern vom oberen Teil der Schiene über die Schultern unter den Axeln zurück zur Schiene und von dort auf der jeweils gleichen Körperseite zu einer Abdominalpelotte führen, von der wiederum untere Gurtabschnitte zum Stützteil führen. Die Abdominalpelotte, die aus zwei Verschlussteilen besteht, ist in einer Höhe angeordnet, in der sie die Bauchatmung nicht behindert.

Dem Anwendungszweck einer solchen Rückenorthese entsprechend ist eine gewisse Einschränkung der Beweglichkeit der Wirbelsäule des Trägers erwünscht. Um dem/der Orthesenträger/in aber auch zu ermöglichen, die Rückenmuskulatur gegen den Widerstand der Schiene zu trainieren, was ein wesentlicher Wirkmechanismus einer derartigen Rückenorthese ist, weisen die bekannten Schienen eine gewisse Flexibilität auf, welche eine eingeschränkte Beugung des Rumpfes gegen einen Widerstand zulässt. In der Praxis hat sich nun die durchgehende Befestigung der Schiene durch die oben beschriebenen Gurte als nachteilig erwiesen, da die Schiene über die Schultern sowie aufgrund der Verlängerung der Bogenlänge des Rückens beim Beugen im Vergleich zum normalen aufrechten Stand, was auf die im Körperinneren liegende neutrale Biegelinie der Wirbelsäule zurückzuführen ist, beim Beugen der Wirbelsäule nach oben gezogen wird. Da bei der auf eine Beugung des Rumpfes folgenden Streckung nicht wieder entsprechende Kräfte in die Richtung zum Boden auf die Schiene wirken und zudem noch die untere Gurtung im allgemeinen die Tendenz hat über die Rundung des Gesäßes nach oben zu verrutschen, verbleibt die einmal ungewollt hochgerutschte Schiene in dieser nicht korrekten Position. Dies ist nicht nur optisch besonders nachteilig, da das obere Ende der Schiene in diesem Fall über die Schultern herausragt, sondern die gesamte Wirkung einer nicht korrekt sitzenden Orthese dieser Art ist in Frage gestellt. Hinzu kommt, dass eine verrutschte Schiene unbequem ist und es zu Druckstellen kommen kann. Des Weiteren hat die bekannte Orthese eine unzureichende Abdominalstützung.

Ein weiterer Nachteil der bekannten Orthese ist es, dass die Schiene in ihrer Gesamtlänge nicht veränderbar ist. Zur Versorgung verschieden großer Patienten ist deshalb die Vorhaltung von verschiedenen Größen besagter Orthese nötig. Außerdem ist es nachteilig, dass trotz der Anfertigung der Schiene in verschiedenen Längen, eine genaue Anpassung oft nicht möglich ist, dann nämlich wenn eine Zwischengröße erforderlich wäre.

Aufgabe der vorliegenden Erfindung ist es, eine Rückenorthese der eingangs genannten Art zu schaffen, die erstens einen gewissen Bewegungsumfang in Beugerichtung des Rumpfes zulässt, welche zweitens eine angemessene Abdominalstützung bewirkt und welche drittens dennoch über eine längere Tragedauer von mehreren Stunden nicht wesentlich am Körper aus ihrer ursprünglichen Position verrutscht.

Zur Lösung dieser Aufgabe sind bei einer Rückenorthese der genannten Art die im Anspruch 1 angegebenen Merkmale vorgesehen.

Durch die erfindungsgemäßen Maßnahmen ist einerseits nach wie vor ein fester Sitz des Stützteils der Rückenorthese gewährleistet und andererseits die Möglichkeit gegeben, den Oberkörper beugend oder streckend zu bewegen, ohne dass die Gefahr besteht, dass das möglicherweise dabei verrutschte Stützteil nicht wieder in seine Ausgangsposition zurückkehrt. Außerdem bleibt dabei die verbesserte Abdominalstützung sowie ein fester Sitz der Rückenorthese erhalten.

Gemäß einer weiteren Ausgestaltung vorliegender Erfindung nach den Merkmalen des Anspruchs 2 ist außerdem eine Schwenkbeweglichkeit des Stützteils gegeben.

Vorteilhafte Ausgestaltungen der vertikalen Beweglichkeit und ggf. der Schwenkbeweglichkeit des Stützteils gegenüber der Bandage ergeben sich aus den Merkmalen eines oder mehrere der Ansprüche 3 bis 5. Hierbei ergibt sich der Vorteil, dass auch bereits auf dem Markt erhältliche Bandagen für eine derartige Rückenorthese verwendet werden können. Besonders einfach ist diese Verwendungsweise dann, wenn die Tasche an der Bandage abnehmbar befestigt ist.

Um den Nachteil der bekannten Rückenorthesen, bei denen das Stützteil nur durch den Hersteller stufenweise für verschiedene Körpergrößen vorgefertigt wird und eine einfache individuelle Anpassung insbesondere der Länge ohne Werkstatteinrichtung auch dem Fachmann nicht möglich ist, zu vermeiden, ist es weiterhin Aufgabe der Erfindung, die Orthese derart zu konstruieren, dass die Anlenkpunkte der Gurte sowie die Länge der Schiene mit einfachen Mitteln von einem Fachmann auch beispielsweise in einer Krankenhausumgebung individuell angepasst werden kann. Zur Lösung dieser Aufgabe sind bei einem weiteren bevorzugten Ausführungsbeispiel vorliegender Erfindung die Merkmale nach Anspruch 6 vorgesehen. Durch diese Maßnahmen ist das Stützteil in einfacherer und kostengünstigerer Weise herstellbar und ohne viel Werkzeug auf die Körpergröße anpassbar.

Um eine Verwindung des Stützteils zu vermeiden, sind die Merkmale nach Anspruch 7 vorgesehen.

Vorteilhafte Ausgestaltungen ergeben sich aus den Merkmalen eines oder mehrere der Ansprüche 8 bis 10, wobei im letzteren Falle eine Anpassung der Rahmenlänge an die Körpergröße besonders einfach ist, weil hierbei die freien Enden der parallelen Profilstäbe ohne weiteres abgelängt werden können, um eine Längenentsprechung zur Oberkörpergröße zu erreichen.

Für eine weitere Stabilisierung des Stützteils sind die Merkmale nach Anspruch 11 vorgesehen. Das Halten der Rückenorthese erfolgt zweckmäßiger Weise entsprechend den im Anspruch 12 vorgesehenen Merkmalen. Die Gurtanordnung für die Rückenorthese ist in vorteilhafter Weise nach den Merkmalen des Anspruchs 13 vorgesehen, wobei das Festlegen der Gurtanordnung am Oberkörper entweder gemäß den Merkmalen des Anspruchs 14 oder gemäß denen des Anspruchs 15 oder gemäß Anspruch 16 vorgenommen werden kann.

Weitere Einzelheiten der Erfindung sind der folgenden Beschreibung zu entnehmen, in der die Erfindung anhand des in der Zeichnung dargestellten Ausführungsbeispieles näher beschrieben und erläutert ist.

### Es zeigen:

- Figur 1: in Vorderansicht eine Rückenorthese gemäß einem bevorzugten Ausführungsbeispiel vorliegender Erfindung;

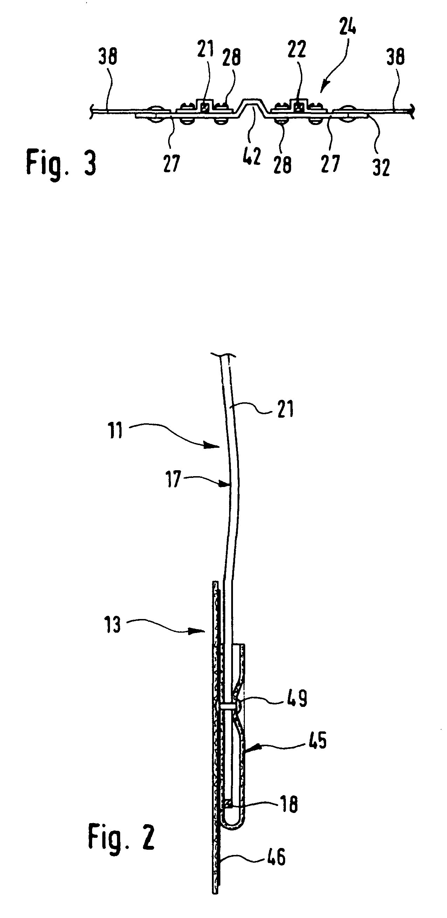

- Figur 2: in vergrößerter Darstellung einen Schnitt längs der Linie II-II der Figur 1;
- Figur 3: in ebenfalls vergrößerter Darstellung einen Schnitt längs der Linie III-III der Figur 1, und
- Figur 4: in schematischer Darstellung eine Möglichkeit der Befestigung der rucksartig getragenen Rückenorthese im Vorderbereich.

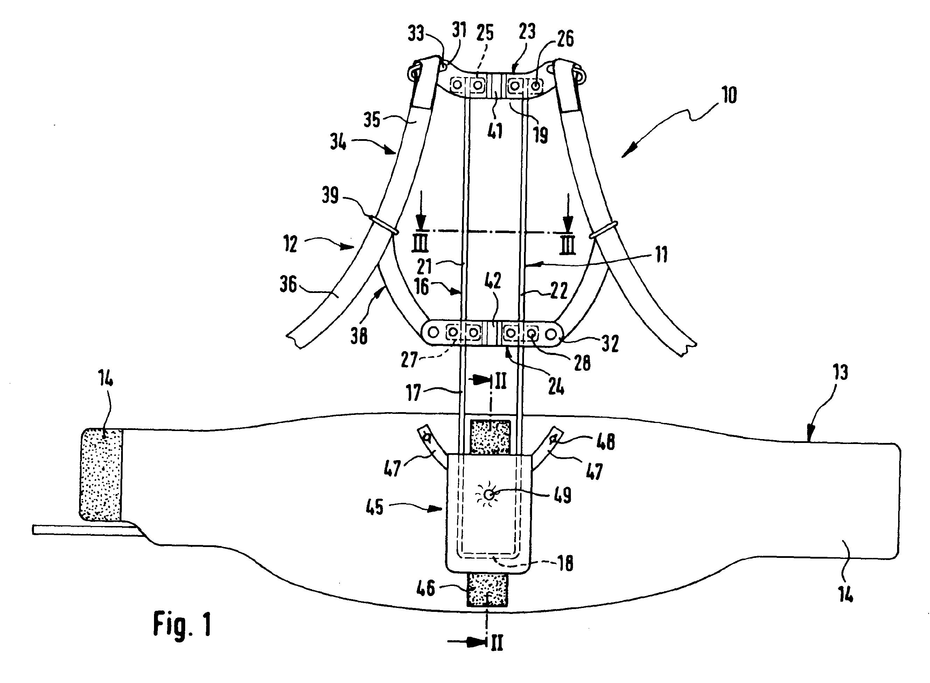

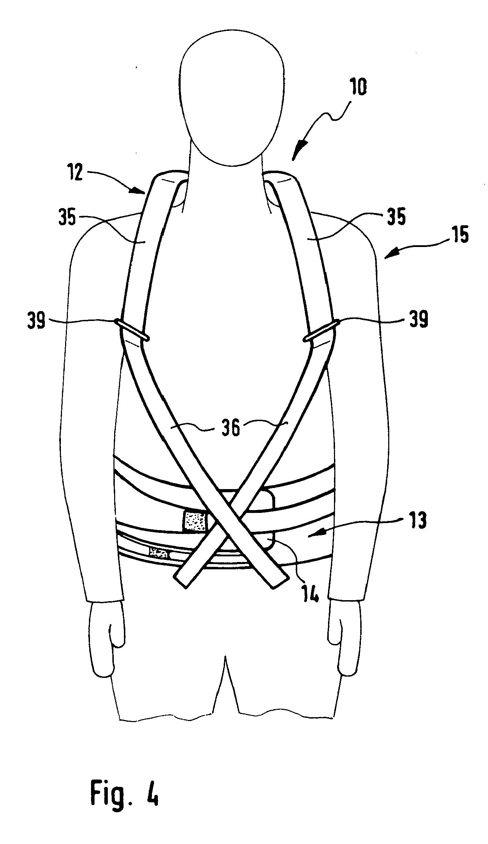

Gemäß der Darstellung der Figur 1 besitzt die Rückenorthese 10 einen Stützteil 11, der sich in nicht im einzelnen dargestellter Weise über im wesentlichen die gesamte Länge der Wirbelsäule eines Trägers 15 (Figur 4) erstreckt und der mittels einer Gurtanordnung 12 rucksackartig auf den Rücken geschnallt getragen wird, sowie eine Bandage 13, die üblicher Bauart sein kann und die vom Träger 15 im Abdominalbereich über den gesamten Unterkörperumfang getragen wird. Dabei ist das Stützteil 11 mit der Bandage 13 trägerrückenseitig in Längsrichtung der Wirbelsäule, d.h. in vertikaler Richtung hin- und herbeweglich verbunden.

Das Stützteil 11 der Rückenorthese 10 besitzt einen U-förmigen Rahmen 16 aus einem U-förmig gebogenen Profilstab 17 mit polygonem, hier quadratischem Querschnitt. Die Anordnung des Rahmens 16 ist derart, dass dessen geschlossenes Ende 18 der Bandage 13 zugewandt ist und dessen offenes Ende 19 nach oben weist.

Die beiden im wesentlichen parallel verlaufenden Profilabschnitte 21 und 22 des U-förmig gebogenen Profilstabes 17 sind an zwei Bereichen durch quer verlaufende Metallplatten 23 bzw. 24 verwindungssteif miteinander verbunden. Davon ist die Metallplatte 23 am oberen offenen Ende 19 des U-förmigen Rahmens 16 angeordnet und derart ausgebildet, dass die beiden Profilstababschnitte 21 und 22 an ihr lösbar festklemmbar gehalten ist. Hierzu kann ein entsprechend profilierter Klemmbügel 25 mit Klemmschrauben 26 Verwendung finden. Die Lösbarkeit des Klemmbügels 25 gegenüber den Profilstababschnitten 21 und 22 ist dazu vorgesehen, je nach Körpergröße des Trägers 15 die Länge des U-förmigen Rahmens 16 und damit des Stützteils 11 dadurch anzupassen, dass die beiden Profilstababschnitte 21 und 22 an ihren freien Enden abgelängt werden können. Die andere Metallplatte 24 ist in einem Bereich zwischen der oberen Metallplatte 23 und dem geschlossenen Ende 18 des Rahmens 16 vorgesehen. Auch die Metallplatte 24 ist über eine entsprechend profilierte Klemmbügel 27 und Klemmschrauben 28 mit den jeweiligen Profilstababschnitten 21 und 22 lösbar festklemmbar verbunden, um sie in eine längenadäquate Position zu bringen. Somit ergibt sich eine formschlüssige und rotationsstabile Verklemmung am offenen Ende bzw. Endbereich des Rahmens 16.

Die beiden Metallplatten 23 und 24 sind außerdem in der Art ausgebildet, dass ihre verlängerten freien Enden ein Halte- bzw. Befestigungsende 31 bzw. 32 für die Gurtanordnung 12 bilden. Hierzu ist die obere Metallplatte 23 an ihren beiden nach oben abgekröpften Enden mit einem geneigt angeordneten Schlitz 33 versehen, durch den jeweils ein oberer Gurt 34 der Gurtanordnung 12 mit seinem oberen Gurtabschnitt 35 längenverstellbar geschleift ist. Die untere bzw. Zwischenmetalleplatte 24 ist an ihren freien verlängerten Enden jeweils mit einem Befestigungsmittel 37, beispielsweise einem Niet, für das eine Ende eines unteren Gurtes 38 der Gurtanordnung 12 versehen, dessen anderes Ende mit einer Schnalle 39versehen ist, durch die der untere Gurtabschnitt 36 des oberen Gurtes 34 geschleift ist. Beide Metallplatten 23 und 24 sind mit einer mittigen Rinne 41, 42 versehen um eventuelle Druckstellen an den Dornfortsätzen der Wirbel des des Orthesenträgers zu vermeiden.

Zur vertikalen Beweglichkeit des Stützteils 11 gegenüber der Bandage 13 ist beim dargestellten Ausführungsbeispiel eine Tasche 45 vorgesehen, die trägerrückenseitig und hierbei innenseitig an der Bandage 13 gehalten ist. Die Tasche 45 ist beim dargestellten Ausführungsbeispiel als Einheit mittels Klettbandverschluss 46 und über zwei seitliche Bänder 47 lösbar verbindbar angeordnet. Die Bänder 47 sind seitlich am dem Stützteil 11 zugewandten Ende angenäht und an ihren freien Enden mit einem Knopf 48 versehen, der in ein Knopfloch der Bandage 13 eingeknöpft ist. Es versteht sich, dass es auch möglich ist, die Tasche 45 unmittelbar auf die Bandage 13 zu befestigen, vorzugsweise anzunähen. Dabei ist es auch möglich, die Tasche 45 in der Weise auszubilden, dass der Tascheninnenraum durch ein Taschenaußenteil und die Innenseite der Bandage 13 gebildet wird.

Die Tasche 45 besitzt außerdem einen quer durch den Tascheninnenraum verlaufenden Anschlagsstift 49, der beispielsweise etwa längsmittig lösbar angeordnet ist und der die Längsbeweglichkeit des unteren geschlossenen Endes 18 des U-förmigen Rahmens 16, das zwischen Taschengrund und Anschlagstift 49 angeordnet ist, begrenzt. Bei dieser Ausgestaltung kann sich also das Stützteil 11 sowohl in vertikaler Richtung, als auch in Schwenkrichtung gegenüber der Bandage 13 innerhalb der Tasche 45 bewegen. Dabei ist die Bewegung nicht nur in einer Richtung, sondern in beiden Richtungen möglich, d.h., dass beispielsweise bei einem Anheben der Schultern das Stützteil 11 relativ zur Bandage 13 mitbewegt wird und beim Absenken der Schultern auch wieder in die Ausgangsposition zurückkehren kann.

Figur 4 zeigt eine Möglichkeit, die Rückenorthese 10 rucksackartig zu tragen und zu halten. Hierzu sind die unteren Gurtabschnitte 36 des oberen Gurtes 34, die durch die Schnallen 39 der unteren Gurte 38 geschleift sind, beispielsweise über Kreuz am lösbaren Verschlussbereich 14 der Bandage 13 beispielsweise über Klettverschlussbänder gehalten.

Es ist aber auch möglich, die durch die Schnallen 39 der unteren Gurte 38 geschleiften unteren Gurtabschnitte 36 der oberen Gurte 34 zurückzuführen und auf den oberen Gurtabschnitten 35 lösbar zu befestigen.

Eine weitere nicht dargestellte Gurtanordnung ist in der Weise möglich, dass die Gurte 38 entfallen und die Gurte 39 zunächst über die Schulter geführt sind, dann unter den Achseln hindurch zurück diagonal über den Rücken verlaufen, um dann über den Beckenkamm der kontralateralen Seite (rechte Schulter, linker Beckenkamm) nach vorne auf die Bandage geführt zu werden, wo er beispielsweise über einen Klettverschluss befestigt wird.

Dies hätte u.U. den Vorteil, dass der natürliche Bewegungsablauf - rechte Schulter vor, linkes Becken zurück - unterstützt wird. Anstatt der Befestigungsmittel 37 an der Zwischenmetallplatte 24 müsste eine Führung für die Gurte angebracht werden, mit der die Höhe des Kreuzungspunktes der Gurte eingestellt werden kann und die Gurte derart geführt werden, dass sie in diesem Bereich gleiten können, ohne sich gegenseitig zu verklemmen.

Es versteht sich ferner, statt einer handelsüblichen Bandage 13, die mit dem Stützteil 11 zur Rückenorthese 10 längs beweglich verbunden wird, eine speziell angefertigte Bandage 13 in Verbindung mit dem Stützteil 11 und der Gurtanordnung 12 für die Rückorthese 10 zu verwenden.

Bei einer weiteren nicht dargestellten Ausführungsform ist das als Rahmen 16 ausgebildete Stützteil 11 in seiner Länge teleskopartig veränderbar und dabei mit seinem unteren geschlossenen Ende 18 an der Bandage festgelegt. Dabei sind die beiden im wesentlichen parallelen Stababschnitte 21 und 22 jeweils zweiteilig entweder nebeneinander gelegt oder ineinander parallel verschiebbar ausgebildet

## Patentansprüche

1. Rückenorthese (10), mit einem sich über einen wesentlichen Teil der Wirbelsäule, vorzugsweise über deren gesamte Länge, erstreckenden Stützteil (11), mit einem Abdominalteil (13) und mit einer Gurtanordnung (12), deren obere Gurtabschnitte mit einem oberen und einem mittleren Bereich des Stützteils (11) verbunden und rucksackträgerartig geführt sind, **dadurch gekennzeichnet, dass** der Abdominalteil zu einer den gesamten unteren Körperteilumfang umschließenden Bandage (13) verlängert ist und dass das wirbelsäulenseitige Stützteil (11) an seinem unteren Ende entweder mit der Bandage (13) in Richtung der Wirbelsäule vertikal beweglich verbunden oder mit der Bandage (13) fest verbunden und in sich teleskopisch vertikal beweglich ist.

2. Rückenorthese nach Anspruch 1, **dadurch gekennzeichnet, dass** das Stützteil (11) mit der Bandage (13) schwenkbeweglich verbunden ist.

3. Rückenorthese nach Anspruch 1 oder 2, **dadurch gekennzeichnet, dass** das Stützteil (11) mit seinem unteren Ende in eine Tasche (45)gesteckt gehalten ist, die mit der Bandage (13) verbunden ist.

4. Rückenorthese nach Anspruch 3, **dadurch gekennzeichnet, dass** die Tasche (45) mit einem die vertikale Bewegung des Stützteils (11) begrenzenden Anschlag (49) versehen ist.

5. Rückenorthese nach Anspruch 3 oder 4, **dadurch gekennzeichnet, dass** die Tasche (45) an der Bandage (13) abnehmbar befestigt ist.

6. Rückenorthese vorzugsweise nach mindestens einem der vorhergehenden Ansprüche, **dadurch gekennzeichnet, dass** das Stützteil (11) durch einen länglich rechteckförmigen Rahmen (16) aus einem metallischen, gebogenen Profilstab (17) gebildet ist.

7. Rückenorthese nach Anspruch 6, **dadurch gekennzeichnet, dass** der Profilstab (17) einen Mehrkant-, vorzugsweise Vierkant-Querschnitt besitzt.

8. Rückenorthese nach Anspruch 6 oder 7, **dadurch gekennzeichnet, dass** der Rahmen (16) U-förmig ausgebildet ist und mit seinem unteren geschlossenen Ende (18) in der Tasche (45) längsbeweglich gehalten ist.

9. Rückenorthese nach Anspruch 8, **dadurch gekennzeichnet, dass** der Rahmen (16) an seinem oberen offenen Ende (19) an einer quer verlaufenden metallischen Platte (23) befestigt ist.

10. Rückenorthese nach Anspruch 9, **dadurch gekennzeichnet, dass** die beiden parallelen Profilstababschnitte (21, 22) am oberen offenen Ende (19) des Rahmens (16) mit der metallischen Platte (23) lösbar verschiebbar festklemmbar ist.

11. Rückenorthese nach mindestens einem der Ansprüche 6 bis 10, **dadurch gekennzeichnet, dass** zwischen der oberen metallischen Platte (23) und der Tasche (45) an der Bandage (13) eine quer verlaufende metallische Zwischenplatte (24) angeordnet ist, an der die parallelen Profilstababschnitte des Rahmens (16) festklemmbar sind.

12. Rückenorthese nach mindestens einem der Ansprüche 6 bis 11, **dadurch gekennzeichnet, dass** die obere (23) und die Zwischenplatte (24) zu beiden Seiten des Rahmens (16) Durchführungs- (33) oder Befestigungsmittel (37) für den oberen und den unteren Gurt (34, 38) der Gurtanordnung (12) besitzen.

13. Rückenorthese nach mindestens einem der vorhergehenden Ansprüche, **dadurch gekennzeichnet, dass** der obere Gurt(34) mit einem oberen Abschnitt (35) durch die obere Metallplatte (23) geschleift und mit einem unteren Abschnitt (36) durch das eine Ende des unteren Gurtes (38) geführt ist, dessen anderes Ende an der Zwischenplatte (24) befestigt ist.

14. Rückenorthese nach Anspruch 13, **dadurch gekennzeichnet, dass** der untere Gurtabschnitt (36) zur Befestigung am oberen Gurtabschnitt (35) desselben oberen Gurtes (34) rückführbar ist.

15. Rückenorthese nach Anspruch 13, **dadurch gekennzeichnet, dass** der untere Gurtabschnitt (36) des oberen Gurtes (34) zur Befestigung zum Abdominalteil der Bandage (13) führbar und dort befestigbar ist.

16. Rückenorthese nach Anspruch 13, dadruch **gekennzeichnet**, dass die oberen Gurtabschnitte (36) unter den Achseln zurückgeführt werden, die beiden Gurte sich im Bereich des Rückenteils in einer Gleitführung überkreuzen und dann jeweils von hinten über die Beckenkämme wieder nach vorne zur Befestigung auf die Bandage (13) geführt werden.
